# FASCICULE DE BREVET EUROPEEN

(11) **EP 2 646 485 B1**
(45) Date de publication et mention de la délivrance du brevet: **25.05.2016**
(21) Numéro de dépôt: 11801787.0
(22) Date de dépôt: 29.11.2011
(51) Int. Cl.: C08F 220/06, C08F 220/60, A61K 8/81, A61K 47/32, A61Q 1/00, A61Q 5/00, A61Q 17/04, A61Q 19/00, C08F 220/28

(54) **NOUVEAUX ÉPAISSISSANTS CATIONIQUES, EXEMPTS D'HUILE ET DE TENSIOACTIFS, PROCÉDÉ POUR LEUR PRÉPARATION ET COMPOSITION EN CONTENANT**
NEUE ÖL- UND TENSIDFREIE KATIONISCHE VERDICKER, VERFAHREN ZU IHRER HERSTELLUNG UND ZUSAMMENSETZUNG DAMIT
NOVEL CATIONIC THICKENERS, FREE OF OIL AND SURFACTANTS, METHOD FOR PREPARING SAME, AND COMPOSITION CONTAINING SAME

(30) Priorité: 01.12.2010 FR 1059950
(43) Date de publication de la demande: 09.10.2013
(73) Titulaire: Société d'Exploitation de Produits pour les Industries Chimiques SEPPIC, 75007 Paris (FR)
(72) Inventeur: BRAUN, Olivier, F-81100 Castres (FR); MALLO, Paul, F-78290 Croissy-sur-Seine (FR)
(74) Mandataire: Conan, Philippe Claude
(86) Numéro de dépôt international: PCT/FR2011/052808
(87) Numéro de publication internationale: WO 2012/072943

(56) Documents cités:
- WO-A1-2009/059887
- WO-A2-2006/123038
- FR-A1- 2 810 882
- FR-A1- 2 900 411

## Description

L'invention a pour objet, de nouveaux épaississants cationiques polymères leur procédé de préparation ainsi que leur utilisation comme agent épaississant et/ou émulsionnant.

L'épaississement des phases aqueuses est en général réalisé en y incorporant des polymères hydrophiles de toutes sortes, qu'ils soient synthétiques ou d'origine naturelle.

Parmi les polymères d'origine naturelle, les gommes xanthane ou de guar sont assez largement utilisées. Ils ont cependant les inconvénients classiques des produits naturels, à savoir une qualité et un prix fluctuant.

Parmi les épaississants synthétiques hydrophiles les plus largement utilisés, il y a les polymères sous forme de poudres ou de latex inverses auto-inversibles. Ils sont mis en oeuvre dans une large gamme de pH et sont souvent bien tolérés par l'homme. De telles compositions sont décrites par exemple dans les brevets américains publiés sous les numéros US 5,004,598, US 6,197,287, US 6,136,305, US 6,346,239 ou dans la demande brevet européen publiée sous le numéro EP 0 503 853. Ces polymères se présentent sous forme de latex ou de poudre. Ces polymères sont anioniques et sont donc essentiellement destinés à épaissir des phases aqueuses contenant les différents constituants classiques que l'on peut trouver dans des formulations topiques de l'industrie cosmétique, dermo-pharmaceutique ou pharmaceutique. On citera notamment des huiles, des tensioactifs (non ioniques ou anioniques) encore appelés émulsionnants, des sels minéraux, des acides faibles. La demande internationale publiée sous le numéro WO 2009/059887 A1 divulgue de copolymères ampholytes à architecture contrôlée et leur utilisation pour contrôler une stabilisation ou une déstabilisation d'un produit dans une composition aqueuse, sous l'impact d'un changement appliqué à ladite composition.

Certaines formulations notamment destinées au soin capillaire contiennent des tensioactifs cationiques et ou des polymères conditionneurs cationiques. Dans ce cas particulier, les épaississants constitués par des polymères anioniques ne sont pas recommandés pour des raisons évidentes pour l'homme de l'art. On utilise de préférence des polymères épaississants cationiques comme ceux décrits dans les brevets américains publiés sous les numéros US 4,806,345 et US 5,100,660.

La demande de brevet français publiée sous le numéro 2 900 411, divulgue des polyélectrolytes amphotères à base d'au moins un monomère anionique et d'au moins un monomère cationique, utilisés dans le traitement de matières minérales, agents réducteurs de la quantité de colloïdes dans la fabrication de papier.

Bien que ces derniers se comportent de manière satisfaisante en milieu acide et qu'ils soient compatibles avec les tensioactifs cationiques, ils perdent néanmoins de leur pouvoir épaississant dans des formulations riches en électrolytes.

Ce problème a été partiellement résolu grâce aux polymères divulgués dans la demande de brevet européen publiée sous le numéro EP 1 449 862.

Néanmoins, lorsqu'ils se présentent sous forme de latex inverses mais aussi sous forme de poudres obtenues par atomisation desdits latex inverses, ces polyélectrolytes comportent des quantités non négligeables non seulement de tensioactifs de bas HLB nécessaires au bon déroulement du procédé de polymérisation en émulsion inverse, mais aussi souvent de tensioactifs de haut HLB dits inverseurs. Or la présence de tels composés peut parfois présenter une gêne pour le formulateur de compositions cosmétiques. De plus la présence d'huiles même en faible quantité comme dans le cas de ces poudres, peut induire un effet négatif sur l'aspect de la formulation finale, aspect, qui joue un rôle certain dans le succès ou dans l'échec commercial de celle-ci.

C'est pourquoi les inventeurs ont cherché à développer des polymères épaississant de type cationique, résistants aux électrolytes exempts de toute présence d'huile ou de tensioactifs comme ceux mentionnés ci-dessus.

Selon un premier aspect, l'invention a pour objet un polyélectrolyte linéaire, branché ou réticulé, issu de la polymérisation pour 100% molaire :
a) d'une proportion molaire supérieure ou égale à 50% et inférieure ou égale à 90% d'unités monomériques issues d'au moins un monomère cationique,
b) d'une proportion molaire supérieure ou égale à 1% et inférieure ou égale à 50% d'unités monomériques issues d'au moins un monomère comportant une fonction acide faible libre partiellement ou totalement salifiée,
c) d'une proportion molaire supérieure ou égale à 0,05% et inférieure ou égale à 5%, molaire, d'unités monomériques issues d'au moins un monomère de formule (I) :

   A-C(=O)-O-[(CH₂-CH(R₁)-O]ₙ-R (I)

   dans laquelle n représente un nombre compris entre 4 et 25, A représente le radical vinyle (CH₂=CH-) ou le radical isoprènyle [CH₂=C(CH₃)-], R₁ représente un atome d'hydrogène, un radical méthyle ou un radical éthyle et R représente un radical aliphatique hydrocarboné, linéaire ou ramifié, saturé ou insaturé, comprenant de 8 à 30 atomes de carbone, et.
d) optionnellement d'une proportion molaire supérieure à 0% et inférieure ou égale à 1% d'un monomère de réticulation diéthylénique ou polyéthylénique.

Par polyélectrolyte branché, on désigne un polyélectrolyte non linéaire qui possède des chaînes pendantes de manière à obtenir, lorsqu'il est mis en solution dans l'eau, un fort état d'enchevêtrement conduisant à des viscosités, à bas gradient de vitesse, très importantes.

Par polyélectrolyte réticulé, on désigne un polyélectrolyte non linéaire se présentant à l'état de réseau tridimensionnel insoluble dans l'eau, mais gonflable à l'eau et conduisant donc à l'obtention d'un gel chimique.

Le polyélectrolyte obtenu par le procédé selon l'invention peut comporter des motifs réticulés et/ou des motifs branchés.

Par monomère cationique, on désigne principalement un monomère aliphatique comportant une fonction ammonium quaternaire et moins une liaison carbone-carbone insaturée. Un tel monomère est généralement disponible sous forme notamment de sels.

Par sels on désigne plus particulièrement les halogénures, tels que les bromures les chlorures ou les iodures desdits monomères à fonction ammonium quaternaire Selon un aspect particulier, l'invention a pour objet le polyélectrolyte tel que défini précédemment, pour lequel les unités monomériques issues d'au moins un monomère cationique, sont issues des sels d'ammonium quaternaires suivants :
- Les sels de N,N,N-triméthyl 3-[(2-méthyl 1-oxo 2-propènyl) amino] propanammonium;
- Les sels de N,N,N-triméthyl 3-[(1-oxo 2-propènyl) amino] propanammonium ;
- Les sels de diallyl diméthylammonium, ou
- Les sels de N,N,N-triméthyl 2-[(2-méthyl 1-oxo 2-propènyl)] éthanammonium ; et plus particulièrement :
   - Le chlorure de N,N,N-triméthyl 3-[(2-méthyl 1-oxo 2-propènyl) amino] propanammonium (MAMPTAC™) ;
   - Le chlorure de N,N,N-triméthyl 3-[(1-oxo 2-propènyl) amino] propanammonium (APTAC™) ;
   - Le chlorure de diallyl diméthylammonium (DADMAC™), ou
   - le chlorure de N,N,N-triméthyl 2-[(2-méthyl 1-oxo 2-propènyl)] éthanammonium (MADQUAT™ MC).

Selon un aspect plus particulier, l'invention a pour objet le polyélectrolyte tel que défini précédemment, pour lequel les unités monomériques issues d'au moins un monomère cationique, sont issues des sels d'ammonium quaternaires suivants :
- Le chlorure de N,N,N-triméthyl 3-[(1-oxo 2-propènyl) amino] propanammonium (APTAC),ou
- Le chlorure de N,N,N-triméthyl 2-[(2-méthyl 1-oxo 2-propènyl)] éthanammonium (MADQUAT™ MC).

Selon un autre aspect particulier de la présente invention, le polyélectrolyte tel que défini ci-dessus est caractérisé en ce que la proportion molaire en unités monomériques issues du ou des monomères cationiques est comprise entre 60,00% et 90,00% molaire.

Par monomère comportant une fonction acide faible, on désigne principalement un monomère comportant une fonction la fonction acide carboxylique.

Selon un autre aspect particulier, l'invention a pour objet le polyélectrolyte tel que défini précédemment, pour lequel les unités monomériques issues d'au moins un monomère comportant une fonction acide faible libre, partiellement ou totalement salifiée, sont issues des acide carboxyliques libres, partiellement ou totalement salifiés suivants :
- L'acide acrylique,
- L'acide méthacrylique,
- L'acide itaconique,
- L'acide maléique, ou
- L'acide 3-méthyl 3-[(1-oxo 2-propènyl) amino] butanoïque.

Selon un aspect plus particulier, il s'agit de l'acide acrylique ou de l'acide méthacrylique.

Selon un aspect tout particulier de la présente invention, ledit monomère possédant une fonction acide faible est l'acide acrylique.

Selon un aspect particulier de la présente invention, le polyélectrolyte tel que défini ci-dessus est caractérisé en ce que la proportion molaire en unités monomériques comportant une fonction acide faible partiellement ou totalement salifiée est comprise entre 5% molaire et 50,00% molaire plus particulièrement inférieure ou égale à 40% et notamment inférieure ou égale à 35%.

Selon un autre aspect particulier, l'invention a pour objet un polyélectrolyte linéaire, branché ou réticulé, dans lequel la proportion molaire en unité d'unités monomériques comportant une fonction acide faible libre, partiellement ou totalement salifiée, issues d'au moins un monomère cationique est supérieure ou égale à 5%.

Pour les monomères comportant une fonction acide faible, le terme salifié indique qu'il s'agit principalement d'un sel de métal alcalin tel que le sel de sodium ou de potassium, ou le sel de bases azotées ou le sel d'ammonium.

Dans la formule (I) telle que définie précédemment, le radical divalent :

-[(CH₂-CH(R₁)-O]ₙ-

Représente notamment :
- Soit une chaîne composée uniquement de groupes éthoxyle (R₁ = H ; n >0),
- Soit une chaîne composée uniquement de groupes propoxyle (R₁ = CH₃ n > 0),
- Soit une chaîne composée uniquement de groupes butoxyle (R₁ = C₂H₅ ; n> 0),
- Soit une chaîne composée d'au moins deux groupes différents choisis parmi les groupes éthoxyle propoxyle et/ou butoxyle.

Lorsque cette chaîne est composée de groupes différents, ils sont distribués tout au long de cette chaîne, de façon séquencée ou aléatoire.

Par radical aliphatique, hydrocarboné linéaire, saturé ou insaturé, comprenant de 8 à 30 atomes de carbone, on désigne plus particulièrement pour R, dans la formule (I) telle que définie précédemment :
- Soit un radical dérivé des alcools primaires linéaires tels que par exemple, ceux dérivés des alcools octylique, pélargonique, décylique, undécylique, undécénylique, laurique, tridécylique, myristylique, pentadécylique, cétylique, heptadécylique, stéarylique, oléylique, linoléylique, nonadécylique, arachidique, béhènylique, érucylique ou I-triacontanoïque. Il s'agit alors des radicaux octyle, nonyle, décyle, undécyle, 10-undécènyle, dodécyle, tridécyle, tétradécyle, pentadécyle, hexadécyle, heptadécyle, octadécyle, 9-octadécènyle, 10,12-octadécadiènyle, 13-docosènyle ou triacontanyle ;
- Soit un radical dérivé des alcools de Guerbet, qui sont des 1-alcanols ramifiés répondant à la formule générale :

   CH₃-(CH₂)ₚ-CH[CH₃-(CH₂)ₚ₋₂]-CH₂OH,

   Dans laquelle p représente un nombre entier compris entre 2 et 14, tels que, par exemple, les radicaux 2-éthyl hexyle, 2-propyl heptyle, 2-butyl octyle, 2-pentyl nonyle, 2-hexyl décyle ou 2-octyl dodécyle ;
- Soit un radical dérivé des isoalcanols répondant à la formule générale :

   CH₃-CH(CH₃)-(CH₂)ₘ-CH₂OH,

   Dans laquelle m représente un nombre entier compris entre 2 et 26 tels que, par exemple, les radicaux 4-méthyl pentyle, 5-méthyl hexyle, 6-méthyl heptyle, 15-méthyl pendadécyle ou 16-méthyl heptadécyle ;
- Soit les radicaux 2-hexyl octyle, 2-octyl décyle ou 2-hexyl dodécyle.

Par radical aliphatique hydrocarboné, linéaire ou ramifié, saturé ou insaturé, comprenant de 8 à 30 atomes de carbone, on désigne plus particulièrement pour R; dans la formule (I) telle que définie précédemment, un radical alkyle comportant de 12 à 22 atomes de carbone.

Selon un aspect tout particulier de la présente invention, ledit monomère de formule (I) telle que définie précédemment, est choisi parmi le méthacrylate de béhènyle pentacosaéthoxylé, composé de formule (I) telle que définie précédemment, dans laquelle R représente le radical docosanyle, A représente le radical isopropènyle, R₁ représente un atome d'hydrogène et n est égal à 25 ; l'acrylate de lauryle tétraéthoxylé, composé qui correspond à la formule (I) telle que définie précédemment, dans laquelle R représente le radical dodécyle, A représente le radical vinyle, R₁ représente un atome d'hydrogène et n est égal à 4, le méthacrylate de stéaryle eicosaéthoxylé, composé de formule (I) telle que définie précédemment, dans laquelle R représente le radical stéaryle, A représente le radical isopropènyle, R₁ représente un atome d'hydrogène et n est égal à 20, ou le méthacrylate de lauryle tétraéthoxylé, composé qui correspond à la formule (I) telle que définie précédemment, dans laquelle R représente le radical dodécyle, A représente le radical isoprènyle, R₁ représente un atome d'hydrogène et n est égal à 4.

L'invention a plus particulièrement pour objet un polyélectrolyte tel que défini précédemment, issu de la polymérisation pour 100% molaire :
a) d'une proportion molaire supérieure ou égale à 60,00% et inférieure ou égale à 90,00% d'unités monomériques issues d'au moins un monomère cationique,
b) d'une proportion molaire supérieure ou égale à 9,95% et inférieure ou égale à 35,00% d'unités monomériques issues d'au moins un monomère comportant une fonction acide faible libre, partiellement ou totalement salifiée, et
c) d'une proportion molaire supérieure ou égale à 0,05% et inférieure ou égale à 5,00% molaire d'unités monomériques issues du composé de formule (I) telle que définie précédemment.

L'invention a aussi tout particulièrement pour objet un polyélectrolyte tel que défini précédemment, issu de la polymérisation pour 100% molaire :
a) d'une proportion molaire supérieure ou égale à 60,00% et inférieure ou égale à 90,00% d'unités monomériques issues du chlorure de N,N,N-triméthyl 2-[( 2-méthyl 1-oxo 2-propènyl)] éthanammonium ,
b) d'une proportion molaire supérieure ou égale à 9,95% et inférieure ou égale à 35% d'unités monomériques issues de l'acide acrylique libre ou partiellement salifié et
c) d'une proportion molaire supérieure ou égale à 0,05% et inférieure ou égale à 5,00% d'unités monomériques issues du méthacrylate de béhènyle pentacosaéthoxylé.

Selon un autre aspect particulier de la présente invention, le polyélectrolyte tel que défini ci-dessus est réticulé.

Selon ce dernier aspect, l'agent de réticulation est choisi notamment parmi les composés diéthyléniques ou polyéthyléniques, et tout particulièrement parmi l'acide diallyloxyacétique ou un des sels et notamment son sel de sodium, le triallylamine, le triméthylol propanetriacrylate, le diméthacrylate d'éthylèneglycol, le diacrylate de diéthylène glycol, le diallylurée ou le méthylène bis(acrylamide).
selon un aspect tout particulier de la présente invention l'agent réticulant mis en oeuvre est le méthylène bis(acrylamide) ou le triméthylol propanetriacrylate.

L'agent de réticulation est alors généralement mis en oeuvre dans la proportion molaire exprimée par rapport aux monomères mis en oeuvre, de 0,005% molaire à 1% molaire, notamment de 0,01 % molaire à 0,2% molaire et plus particulièrement de 0,01% molaire à 0,1% molaire.

L'invention a aussi tout particulièrement pour objet un polyélectrolyte tel que défini précédemment, issu de la polymérisation pour 100% molaire :
a) d'une proportion molaire supérieure ou égale à 60,00% et inférieure ou égale à 90,00% d'unités monomériques issues du chlorure de N,N,N-triméthyl 2-[(2-méthyl 1-oxo 2-propènyl)] éthanammonium ,
b) d'une proportion molaire supérieure ou égale à 9,90% et inférieure ou égale à 35% d'unités monomériques issues de l'acide acrylique libre ou partiellement salifié,
c) d'une proportion molaire supérieure ou égale à 0,05% et inférieure ou égale à 5,00% d'unités monomériques issues du méthacrylate de béhènyle pentacosaéthoxylé. Et
d) d'une proportion supérieure ou égale à 0,005% molaire et inférieure à 1% molaire de triméthylol propanetriacrylate.

Selon un autre aspect de la présente invention, l'invention a pour objet un procédé de préparation d'un polyélectrolyte tel que défini précédemment, comprenant les étapes successives suivantes :
Une étape a) de préparation d'un mélange réactionnel comprenant dans les proportions molaires souhaitées et dans un solvant (S), d'unités monomériques issues d'au moins un monomère cationique, d'unités monomériques issues d'au moins un monomère comportant une fonction acide faible libre partiellement ou totalement salifiée, d'unités monomériques issues d'au moins un monomère de formule (I) telle que définie précédemment et, si nécessaire ou si désiré de l'agent réticulant et/ ou des autres additifs, ledit solvant (S) étant :
   - Soit une cétone de formule (II) : 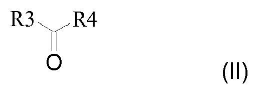 dans laquelle R3 et R4 identiques ou différents, représentent indépendamment l'un de l'autre un radical méthyle, un radical éthyle, un radical isopropyle ou un radical isobutyle ;
   - Soit un mélange consistant en, pour 100% molaire :
      - de l'eau en une proportion supérieure à 0% molaire et inférieure ou égale à 25% molaire ; et
      - une cétone de formule (II) telle que définie ci-dessus, en une proportion supérieure ou égal à 75% molaire et inférieure à 100% ;
Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte linéaire.

Selon un autre aspect particulier de la présente invention, dans l'étape b) du procédé tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis(isobutyronitrile) ou encore les dérivés azoïques.

Selon un autre aspect particulier de la présente invention, dans l'étape b) du procédé tel que défini précédemment, la réaction de polymérisation est amorcée par un couple oxydoréducteur tel qu'un couple oxydoréducteur générateur d'ions hydrogénosulfite (HSO₃) comme le couple hydroperoxyde de cumène - métabisulfite de sodium (Na₂S₂O₅) ou le couple hydroperoxyde de cumène-chlorure de thionyle (SOCl₂) à une température inférieure ou égale à 20°C, si désiré accompagné d'un agent co-initiateur de polymérisation tel que par exemple l'azo-bis(isobutyronitrile), le dilaurylperoxyde ou le persulfate de sodium , puis conduite de manière quasi-adiabatique.

Selon un autre aspect particulier de la présente invention, dans l'étape b) du procédé tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis(isobutyronitrile) ou encore les dérivés azoïques.

Le procédé tel que défini ci-dessus peut comprendre en outre une étape c) d'isolation dudit précipité obtenu à l'étape b) par séparation d'avec ledit solvant (S), puis si nécessaire ou si désiré, une étape d) de séchage dudit précipité résultant de l'étape c.

Selon un autre aspect particulier de la présente invention, dans l'étape c) du procédé tel que défini précédemment, la séparation du précipité obtenu dudit solvant organique est effectué par filtration.

Selon un autre aspect particulier, l'invention a pour objet un procédé tel que défini ci-dessus, dans lequel ledit solvant (S) est :
- Soit une cétone choisie parmi le propane-2-one, le butane-2-one, le pentane-2-one, le 3-méthyl butane-2-one, le 3-éthyl pentane-20-one ou le 4-méthyl pentane-2-one ;
- Soit un mélange consistant en pour 100% molaire :
   - de l'eau en une proportion supérieure à 0% molaire et inférieure ou égale à 10% molaire, de préférence inférieure ou égale à 5% molaire ; et
   - une cétone choisie parmi le propane-2-one, le butane-2-one, le pentane-2-one, le 3-méthyl butane-2-one, le 3-éthyl pentane-2-one ou le 4-méthyl pentane-2-one de formule (II) telle que définie précédemment, en une proportion molaire supérieure ou égale à 90% molaire, de préférence supérieure ou égale à 95% molaire et inférieure à 100% molaire.

Selon un aspect tout particulier, l'invention a pour objet le procédé tel que défini ci-dessus, dans lequel ledit solvant (S) est soit de l'acétone, soit un mélange eau-acétone en un ratio molaire eau/acétone supérieur à 0 et inférieur ou égal à 5/95.

Grâce à son caractère cationique, le polyélectrolyte objet de la présente invention, est avantageusement utilisé comme épaississant et/ou comme émulsionnant dans des compositions cosmétiques ou pharmaceutiques destinées au soin et/ou au conditionnement des cheveux.

C'est pourquoi selon un autre aspect, l'invention a pour objet, l'utilisation du polyélectrolyte tel que défini précédemment, comme agent épaississant et/ou comme agent émulsionnant dans des compositions cosmétiques ou pharmaceutiques et plus particulièrement celles destinées au soin et/ou au conditionnement des cheveux.

Le polyélectrolyte objet de la présente invention peut être formulé dans des formules cosmétiques ou pharmaceutiques comme des mousses, des gels, des lotions, des sprays, des shampoings, des conditionneurs, des lotions pour les mains et le corps, des écrans solaires, et plus généralement dans des produits de soin.

Dans le cas du traitement ou de l'entretien du cheveu, de telles compositions cosmétiques ou pharmaceutiques se présentent habituellement sous forme de shampoings d'émulsions, de microémulsions et notamment dans le cas des conditionneurs, d'émulsions vaporisables.

Selon un dernier aspect l'invention a pour objet, une composition cosmétique ou pharmaceutique caractérisée en en ce qu'elle contient comme agent émulsionnant et/ou épaississant, une quantité efficace du polyélectrolyte tel que définie précédemment.

Par quantité efficace, on entend une proportion pondérale comprise entre environ 0,1% et environ 5% en poids du polyélectrolyte tel que définie précédemment.

Les exemples suivants illustrent la présente invention sans toutefois la limiter.

### Exemple 1 : Préparation d'un terpolymère acide acrylique/MADQUAT/BEM-25

### partiellement salifié sous forme de sel de sel sodium (Composé 1)

a) On ajoute successivement dans un bêcher contenant 400 g d'acétone :
   - 6,63 g de d'acide acrylique ;
   - 7,75 g d'hydrogénocarbonate de sodium
   - 242,4g d'une solution commerciale à 75% de chlorure de N,N,N-triméthyl 2-[(1-oxo 2-propènyl) amino] éthanammonium (MADQUAT MC 75™) ;
   - 0,58 g de méthacrylate de béhènyle pentacosaéthoxylé (BEM-25).
b) L'ensemble est laissé sous agitation et barbotage d'azote pendant 45 minutes, chauffé jusqu'à 55 °C puis additionné de 0,75 g de peroxyde de dilauryle pour amorcer la réaction de polymérisation.
   Après deux de reflux le milieu réactionnel est refroidi.
c) Le précipité obtenu est filtré puis séché sous vide. On obtient le polymère attendu sous forme de poudre.

Les propriétés viscosimétriques de la poudre de polymère obtenue sont les suivantes
Viscosité à 25°C d'une dispersion aqueuse comprenant 2 % massique de la poudre obtenue : 25 280 mPa.s (Brookfield RVT, M6 V5)
Viscosité d'une dispersion aqueuse comprenant 2 % massique de la poudre obtenue et 0,1 % massique de chlorure de sodium : 11 800 mPas (Brookfield RVT, M3 V5).

### Exemple 2 : Préparation d'un terpolymère acide acrylique/MADQUAT/BEM-25 partiellement salifié sous forme de sel de sel sodium réticulé au triméthylol Propanetriacrylate (Composé 2)

a) On ajoute successivement dans un bêcher contenant 400 g d'acétone :
   - 6,63 g de d'acide acrylique ;
   - 7,75 g d'hydrogénocarbonate de sodium
   - 242,4g d'une solution commerciale à 75% de chlorure de N,N,N-triméthyl 2-[(1-oxo 2-propènyl) amino] éthanammonium (MADQUAT MC 75™) ;
   - 0,58 g de méthacrylate de béhènyle pentacosaéthoxylé (BEM-25), et
   - 0,16 g de triméthylol propane triacrylate.
b) L'ensemble est laissé sous agitation et barbotage d'azote pendant 45 minutes, chauffé jusqu'à 55 °C puis additionné de 0,75 g de peroxyde de dilauryle pour amorcer la réaction de polymérisation.
   Après deux de reflux le milieu réactionnel est refroidi.
c) Le précipité obtenu est filtré puis séché sous vide. On obtient le polymère attendu sous forme de poudre.

Les propriétés viscosimétriques de la poudre de polymère obtenue sont les suivantes:
Viscosité à 25°C d'une dispersion aqueuse comprenant 2 % massique de la poudre obtenue : 19 920 mPa.s (Brookfield RVT, M6 V5)
Viscosité d'une dispersion aqueuse comprenant 2 % massique de la poudre obtenue et 0,1 % massique de chlorure de sodium : 7 740 mPas (Brookfield RVT, M3 V5).

### Exemple 3 : Soin antistress pour cheveux

**Formule**

| Phase A | |
|---|---|
| Eau : | QSP 100% |
| Gomme xanthane | 0,50 % |

| Phase B | |
|---|---|
| SEPICAP™MP: | 3,00 % |

| Phase C | |
|---|---|
| Composé 1 : | 4,00 % |

| Phase D | |
|---|---|
| Butylène Glycol : | 5,00 % |
| LANOL™ 99 : | 5,00 % |
| SEPICIDE™ HB : | 0,30 % |
| SEPICIDE™ CI : | 0,20 % |
| Parfum | 0,20 % |

### Mode Opératoire

Disperser la gomme xanthane dans l'eau avec une défloculeuse. Ajouter ensuite SEPICAP™ MP, puis le composé 1 ; le disperser puis ajouter les ingrédients de la phase D.

### Exemple 4 : Masque crème restructurant pour cheveux stressés et fragilisés

**Formule**

| Phase A | |
|---|---|
| MONTANOV™ 82 : | 3,00 % |
| LANOL™ P : | 6,00 % |
| AMONYL™DM : | 1,00 % |
| Isononanoate d'isostéaryle : | 5,00 % |
| Composé 2 : | 2,50 % |

| Phase B | |
|---|---|
| Eau : | QSP 100% |

| Phase C | |
|---|---|
| SEPICAP™ MP : | 3,00 % |
| SEPICIDE™ HB : | 0,30 % |
| SEPICIDE™ CI : | 0, 20 % |

### Mode Opératoire

Fondre la phase A à 75°C. Chauffer la phase B à 75°C. Emulsionner A dans B. Vers 40°C introduire les constituants de la phase C.

### Exemple 5 : Gel purifiant pour visage

**Formule**

| Phase A | |
|---|---|
| MONTALINE™ C 40 : | 7,00 % |
| Base nacrante 2078 : | 5,00 % |
| Composé 1 : | 2,00% |

| Phase B | |
|---|---|
| Eau : | QSP 100% |

### Exemple 6 : Shampoing colorant

**Formule**

| Phase A | |
|---|---|
| MONTALINE™ C 40 : | 15,00 % |
| Cocamphoacétate disodé : | 5,00 % |
| Cetrimonium chloride : | 1,00 % |
| SEPIPERL™ N : | 3,00 % |
| Composé 1 : | 3,00 % |

| Phase B | |
|---|---|
| Couleur | QSP |
| Eau | QSP 100 % |

### Exemple 7 : Emulsion fluide à pH alcalin

| | |
|---|---|
| MARCOL™ 82 : | 5,0% |
| Hydroxyde sodium : | 10,0% |
| Eau : | q.s.p. 100% |
| Composé 2 : | 1,5% |

### Exemple 8 : Masque crème "rince off" restructurant pour cheveux stressés et fragilisés

| | |
|---|---|
| KETROL™T : | 0,5% |
| PECOSIL™ SPP50 : | 0,75% |
| N-cocoyl aminoacides : | 0,70% |
| Butylèneglycol : | 3,0% |
| Composé 1 : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™ 99 : | 5,0% |
| SEPICIDE™ HB : | 0,3% |
| SEPICIDE™CI : | 0,2% |
| Parfum : | 0,2% |
| Eau : | qsp. 100% |

### Exemple 9 : Lotion capillaire

| | |
|---|---|
| Butylène glycol : | 3,0% |
| Composé 1 : | 3,0% |
| SIMULSOL™1293 : | 3,0% |
| Acide lactique : | qs. pH = 6 |
| SEPICIDE™ HB : | 0,2% |
| SEPICIDE™CI : | 0,3% |
| Parfum : | 0,3% |
| Eau : | qs. 100% |

### Exemple 10 : Shampooing protecteur et relaxant

| | |
|---|---|
| Amonyl™ 675 SB : | 5,0% |
| Sodium lauroyl éther sulfate à 28% : | 35,0% |
| Composé 2 : | 3,0% |
| SEPICIDE™ HB: | 0,5% |
| SEPICIDE™CI : | 0,3% |
| Hydroxyde de sodium : | q.s. pH = 7,2 |
| Parfum : | 0,3% |
| Colorant (FDC bleu 1/jaune 5) : | q.s. |
| Eau : | qsp. 100% |

### Exemple 11 : Protecteur "leave-on" ; Soin antistress pour cheveux

| | |
|---|---|
| KETROL™T : | 0,5% |
| Mélange de cocoyl aminoacides : | 3,0% |
| Butylèneglycol : | 5,0% |
| DC 1501 : | 5,0% |
| Composé 1 : | 4,0% |
| SEPICIDE™ HB: | 0,5% |
| SEPICIDE™CI : | 0,3% |
| Parfum : | 0,3% |
| Eau : | qsp.100 |

Les définitions des produits commerciaux utilisés dans les exemples sont les suivantes:
MONTALINE™ C40 : (cocamoniumcarbamoyl chloride) commercialisé par SEPPIC. SEPIPERL™ N : (cocoyl glucoside *1* cocoyl alcohol) commercialisé par SEPPIC. AMONYLT^{M} DM : (Quatemium 82) commercialisé par SEPPIC.
SEPICAP™ MP : (Sodium cocoyl amino acids *1* potassium dimethicone copolyol panthenyl phosphate) commercialisé par SEPPIC.
SIMULSOL™ 1293 est de l'huile de castor hydrogénée et éthoxylée, avec un indice d'éthoxylation égal à 40, commercialisé par la société SEPPIC.
KETROL™T est de la gomme de xanthane commercialisée par la société KELCO.
LANOL™ 99 est de l'isononanoate d'isononyle commercialisé par la société SEPPIC.
DC1501 est un mélange de cyclopentasiloxane et de diméthiconol commercialisé par la société DOW CHEMICAL.
MONTANOV™ 82 est un agent émulsionnant à base d'alcool cétéarylique et de cocoylglucoside.
Le SEPICIDE™ Cl, imidazolidine urée, est un agent conservateur commercialisé par la société SEPPIC.
Le SEPICIDE™HB, qui est un mélange de phénoxyéthanol, de méthylparaben, d'éthylparaben, de propylparaben et de butylparaben, est un agent conservateur commercialisé par la société SEPPIC.
Le LANOL™ P est un additif à effet stabilisant commercialisé par la société SEPPIC.

## Revendications

1. Polyélectrolyte linéaire, branché ou réticulé, issu de la polymérisation pour 100% molaire :
a) d'une proportion molaire supérieure ou égale à 50% et inférieure ou égale à 90% d'unités monomériques issues d'au moins un monomère cationique,
b) d'une proportion molaire supérieure ou égale à 1% et inférieure ou égale à 50% d'unités monomériques issues d'au moins un monomère comportant une fonction acide faible libre partiellement ou totalement salifiée,
c) d'une proportion molaire supérieure ou égale à 0,05% et inférieure ou égale à 5%, molaire, d'unités monomériques issues d'au moins un monomère de formule (I) :
A-C(=O)-O-[(CH₂-CH(Rₗ)-O]ₙ-R (I)
dans laquelle n représente un nombre compris entre 4 et 25, A représente le radical vinyle (CH₂=CH-) ou le radical isopropènyle [CH₂=C(CH₃)-]
, R₁ représente un atome d'hydrogène, un radical méthyle ou un radical éthyle et R représente un radical aliphatique hydrocarboné, linéaire ou ramifié, saturé ou insaturé, comprenant de 8 à 30 atomes de carbone, et.
d) optionnellement d'une proportion molaire supérieure à 0% et inférieure ou égale à 1% d'un monomère de réticulation diéthylénique ou polyéthylénique.

2. Polyélectrolyte tel que défini à la revendication 1, pour lequel les unités monomériques issues d'au moins un monomère cationique, sont issues des sels d'ammonium quaternaires suivants :
- Les sels de N,N,N-triméthyl 3-[(2-méthyl 1-oxo 2-propènyl) amino] propanammonium;
- Les sels de N,N,N-triméthyl 3-[(1-oxo 2-propènyl) amino] propanammonium ;
- Les sels de diallyl diméthylammonium, ou
- Les sels de N,N,N-triméthyl 2-[(2-méthyl 1-oxo 2-propènyl)] éthanammonium ;

3. Polyélectrolyte tel que défini à la revendication 2, pour lequel les unités monomériques issues d'au moins un monomère cationique, sont issues des sels d'ammonium quaternaires suivants :
- le chlorure de N,N,N-triméthyl 3-[(2-méthyl 1-oxo 2-propènyl) amino] propanammonium;
- le chlorure de N,N,N-triméthyl 3-[(1-oxo 2-propènyl) amino] propanammonium ;
- le chlorure de diallyl diméthylammonium ;
- le chlorure de N,N,N-triméthyl 2-[(1-oxo 2-propènyl)] éthanammonium ; ou
- le chlorure de N,N,N-triméthyl 2-[(2-méthyl 1-oxo 2-propènyl)] éthanammonium.

4. Polyélectrolyte tel que défini à la revendication 3, pour lequel les unités monomériques issues d'au moins un monomère cationique, sont issues des sels d'ammonium quaternaires suivants :
- Le chlorure de N,N,N-triméthyl 3-[(1-oxo 2-propènyl) amino] propanammonium, ou
- Le chlorure de N,N,N-triméthyl 2-[(2-méthyl 1-oxo 2-propènyl)] éthanammonium.

5. Polyélectrolyte tel que défini à l'une quelconque des revendications 1 à 4, pour lequel les unités monomériques issues d'au moins un monomère comportant une fonction acide faible libre, partiellement ou totalement salifiée, sont issues des acide carboxyliques libres, partiellement ou totalement salifiés suivants :
- L'acide acrylique,
- L'acide méthacrylique,
- L'acide itaconique,
- L'acide maléique, ou
- L'acide 3-méthyl 3-[(1-oxo 2-propènyl) amino] butanoïque.

6. Polyélectrolyte tel que défini à l'une quelconque des revendications 1 à 5, pour laquelle le composé de formule (I) telle que définie précédemment, est choisi parmi :
- Le méthacrylate de béhènyle pentacosaéthoxylé ;
- L'acrylate de lauryle tétraéthoxylé ;
- Le méthacrylate de stéaryle eicosaéthoxylé ; ou
- Le méthacrylate de lauryle tétraéthoxylé ;

7. Polyélectrolyte tel que défini à l'une quelconque des revendications 1 à 6, issu de la polymérisation pour 100% molaire :
a) d'une proportion molaire supérieure ou égale à 60,00% et inférieure ou égale à 90,00% d'unités monomériques issues d'au moins un monomère cationique,
b) d'une proportion molaire supérieure ou égale à 9,95% et inférieure ou égale à 35,00% d'unités monomériques issues d'au moins un monomère comportant une fonction acide faible libre, partiellement ou totalement salifiée, et
c) d'une proportion molaire supérieure ou égale à 0,05% et inférieure ou égale à 5,00% molaire d'unités monomériques issues du composé de formule (I) telle que définie précédemment.

8. Polyélectrolyte tel que défini à la revendication 7, issu de la polymérisation pour 100% molaire :
a) d'une proportion molaire supérieure ou égale à 60,00% et inférieure ou égale à 90,00% d'unités monomériques issues du chlorure de N,N,N-triméthyl 2-[(2-méthyl 1-oxo 2-propènyl)] éthanammonium,
b) d'une proportion molaire supérieure ou égale à 9,95% et inférieure ou égale à 35% d'unités monomériques issues de l'acide acrylique libre ou partiellement salifié et
c) d'une proportion molaire supérieure ou égale à 0,05% et inférieure ou égale à 5,00% d'unités monomériques issues du méthacrylate de béhènyle pentacosaéthoxylé.

9. Polyélectrolyte tel que défini à l'une quelconque des revendications 1 à 8, **caractérisé en ce qu'**il est réticulé.

10. Polyélectrolyte tel que défini à la revendication 9, issu de la polymérisation pour 100% molaire :
a) d'une proportion molaire supérieure ou égale à 60,00% et inférieure ou égale à 90,00% d'unités monomériques issues du chlorure de N,N,N-triméthyl 2-[(2-méthyl 1-oxo 2-propènyl)] éthanammonium ,
b) d'une proportion molaire supérieure ou égale à 9,90% et inférieure ou égale à 35% d'unités monomériques issues de l'acide acrylique libre ou partiellement salifié,
c) d'une proportion molaire supérieure ou égale à 0,05% et inférieure ou égale à 5,00% d'unités monomériques issues du méthacrylate de béhènyle pentacosaéthoxylé. Et
d) d'une proportion molaire supérieure ou égale à 0,005% et inférieure à 1% de triméthylol propanetriacrylate.

11. Procédé de préparation d'un polyélectrolyte tel que défini à l'une des revendications 1 à 10 comprenant les étapes successives suivantes :
Une étape a) de préparation d'un mélange réactionnel comprenant dans les proportions molaires souhaitées et dans un solvant (S), d'unités monomériques issues d'au moins un monomère cationique, d'unités monomériques issues d'au moins un monomère comportant une fonction acide faible libre partiellement ou totalement salifiée, et optionnellement d'unités monomériques issues d'au moins un monomère de formule (I) telle que définie précédemment et, si nécessaire ou si désiré de l'agent réticulant et/ ou des autres additifs, ledit solvant (S) étant :
- Soit une cétone de formule (II) : 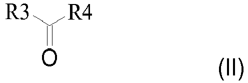 dans laquelle R3 et R4 identiques ou différents, représentent indépendamment l'un de l'autre un radical méthyle, un radical éthyle, un radical isopropyle ou un radical isobutyle ;
- Soit un mélange consistant en, pour 100% molaire :
- de l'eau en une proportion supérieure à 0% molaire et inférieure ou égale à 25% molaire ; et
- une cétone de formule (II) telle que définie ci-dessus, en une proportion supérieure ou égal à 75% molaire et inférieure à 100% ;
Une étape b) au cours de laquelle la réaction de polymérisation est amorcée par introduction dans ledit mélange réactionnel préparé à l'étape a), d'un initiateur de radicaux libres, puis est laissée se dérouler jusqu'à sa conclusion, pour obtenir un précipité dudit polyélectrolyte linéaire.

12. Utilisation du polyélectrolyte telle que défini à l'une des revendications 1 à 10, comme agent épaississant et/ou comme agent émulsionnant dans des compositions cosmétiques ou pharmaceutiques et plus particulièrement celles destinées au soin et/ou au conditionnement des cheveux.

13. Composition cosmétique ou pharmaceutique caractérisée en en ce qu'elle contient comme agent émulsionnant et/ou épaississant, une quantité efficace du polyélectrolyte tel que défini à l'une quelconque des revendications 1 à 10.

## Patentansprüche

1. Linearer, verzweigter oder netzförmiger Polyelektrolyt aus der Polymerisation für 100 Molprozent:
a) eines Molverhältnisses größer oder gleich 50 % und kleiner oder gleich 90 % von Monomereinheiten aus mindestens einem kationischen Monomer,
b) eines Molverhältnisses größer oder gleich 1 % und kleiner oder gleich 50 % von Monomereinheiten aus mindestens einem Monomer, umfassend eine schwache, freie, teilweise oder vollständig salzhaltige Säurefunktion,
c) eines Molverhältnisses größer oder gleich 0,05 % und kleiner oder gleich 5 Molprozent von Monomereinheiten aus mindestens einem Monomer mit der folgenden Formel (I):
A-C(=O)-O-[(CH₂-CH(R_{I})-O]ₙ-R (I)
wobei n eine Zahl zwischen 4 und 25 darstellt, A das Vinylradikal (CH₂=CH-) oder das Isopropenylradikal [CH₂=C(CH₃)-] darstellt, R₁ ein Wasserstoffatom, ein Methylradikal oder ein Ethylradikal darstellt, und R ein lineares oder verzweigtes, gesättigtes oder ungesättigtes aliphatisches Kohlenwasserstoffradikal darstellt, umfassend 8 bis 30 Kohlenstoffatome, und
d) optional eines Molverhältnisses größer 0 % und kleiner oder gleich 1 % eines Diethylen- oder Polyethylen-Vernetzungsmonomers.

2. Polyelektrolyt, wie in Anspruch 1 definiert, wobei die Monomereinheiten aus mindestens einem kationischen Monomer aus den folgenden quaternären Ammoniumsalzen stammen:
- den N,N,N-trimethyl 3-[(2-methyl 1-oxo 2-propenyl) amino] propanammoniumsalzen;
- den N,N,N-trimethyl 3-[(1-oxo 2-propenyl) amino] propanammoniumsalzen;
- den Diallyldimethylammoniumsalzen, oder
- den N,N,N-trimethyl 2-[(2-methyl 1-oxo 2-propenyl)] ethanammoniumsalzen;

3. Polyelektrolyt, wie in Anspruch 2 definiert, wobei die Monomereinheiten aus mindestens einem kationischen Monomer aus den folgenden quaternären Ammoniumsalzen stammen:
- dem N,N,N-trimethyl 3-[(2-methyl 1-oxo 2-propenyl) amino] propanammoniumchlorid;
- dem N,N,N-trimethyl 3-[(1-oxo 2-propenyl) amino] propanammoniumchlorid;
- dem Diallyldimethylammoniumchlorid;
- dem N,N,N-trimethyl 2-[(1-oxo 2-propenyl)] ethanammoniumchlorid; oder
- dem N,N,N-trimethyl 2-[(2-methyl 1-oxo 2-propenyl)] ethanammoniumchlorid.

4. Polyelektrolyt, wie in Anspruch 3 definiert, wobei die Monomereinheiten aus mindestens einem kationischen Monomer aus den folgenden quaternären Ammoniumsalzen stammen:
- dem N,N,N-trimethyl 3-[(1-oxo 2-propenyl) amino] propanammoniumchlorid, oder
- dem N,N,N-trimethyl 2-[(2-methyl 1-oxo 2-propenyl)] ethanammoniumchlorid.

5. Polyelektrolyt, wie in einem der Ansprüche 1 bis 4 definiert, wobei die Monomereinheiten aus mindestens einem kationischen Monomer, umfassend eine schwache, freie, teilweise oder vollständig salzhaltige Säurefunktion, aus den folgenden freien, teilweise oder vollständig salzhaltigen Karbonsäuren stammen:
- Acrylsäure
- Methacrylsäure,
- Itaconsäure
- Maleinsäure, oder
- 3-Methyl 3-[(1-oxo 2-propenyl) amino] butansäure.

6. Polyelektrolyt, wie in einem der Ansprüche 1 bis 5 definiert, wobei die Verbindung mit der Formel (I), wie oben definiert, ausgewählt ist aus:
- pentacosaethoxyliertem Behenylmethacrylat;
- tetraethoxyliertem Laurylacrylat;
- eicosaethoxyliertem Stearylmethacrylat; oder
- tetraethoxyliertem Laurylmethacrylat.

7. Polyelektrolyt, wie in einem der Ansprüche 1 bis 6 definiert, aus der Polymerisation für 100 Molprozent:
a) eines Molverhältnisses größer oder gleich 60,00 % und kleiner oder gleich 90,00 %von Monomereinheiten aus mindestens einem kationischen Monomer,
b) eines Molverhältnisses größer oder gleich 9,95 % und kleiner oder gleich 35,00 % von Monomereinheiten aus mindestens einem Monomer, umfassend eine schwache, freie, teilweise oder vollständig salzhaltige Säurefunktion, und
c) eines Molverhältnisses größer oder gleich 0,05 % und kleiner oder gleich 5,00 Molprozent von Monomereinheiten aus der Verbindung mit der Formel (I), wie oben definiert.

8. Polyelektrolyt, wie in Anspruch 7 definiert, aus der Polymerisation für 100 Molprozent:
a) eines Molverhältnisses größer oder gleich 60,00 % und kleiner oder gleich 90,00 % von Monomereinheiten aus dem N,N,N-trimethyl 2-[(2-methyl 1-oxo 2-propenyl)] ethanammoniumchlorid,
b) eines Molverhältnisses größer oder gleich 9,95 % und kleiner oder gleich 35 % von Monomereinheiten aus der freien oder teilweise salzhaltigen Acrylsäure und
c) eines Molverhältnisses größer oder gleich 0,05 % und kleiner oder gleich 5,00 % von Monomereinheiten aus dem pentacosaethoxyliertem Behenylmethacrylat.

9. Polyelektrolyt, wie in einem der Ansprüche 1 bis 8 definiert, **dadurch gekennzeichnet, dass** er netzartig ist.

10. Polyelektrolyt, wie in Anspruch 9 definiert, aus der Polymerisation für 100 Molprozent:
a) eines Molverhältnisses größer oder gleich 60,00 % und kleiner oder gleich 90,00 % von Monomereinheiten aus dem N,N,N-trimethyl 2-[(2-methyl 1-oxo 2-propenyl)] ethanammoniumchlorid,
b) eines Molverhältnisses größer oder gleich 9,90 % und kleiner oder gleich 35 % von Monomereinheiten aus der freien oder teilweise salzhaltigen Acrylsäure,
c) eines Molverhältnisses größer oder gleich 0,05 % und kleiner oder gleich 5,00 % von Monomereinheiten aus pentacosaethoxyliertem Behenylmethacrylat, und
d) eines Molverhältnisses größer oder gleich 0,005 % und kleiner oder gleich 1 % von Trimethylolpropantriacrylat.

11. Verfahren zur Herstellung eines Polyelektrolyts, wie in einem der Ansprüche 1 bis 10 definiert, umfassend die folgenden aufeinander folgenden Schritte:
einen Schritt a) des Herstellens einer Reaktionsmischung, umfassend in den gewünschten molaren Verhältnissen und in einem Lösemittel (S) Monomereinheiten aus mindestens einem kationischen Monomer Monomereinheiten aus mindestens einem Monomer,
umfassend eine schwache, freie, teilweise oder vollständig salzhaltige Säurefunktion, und optional Monomereinheiten aus mindestens einem Monomer mit der Folgenden Formel (I), wie oben definiert, und, falls erforderlich oder falls gewünscht, ein Vernetzungsmittel und/oder andere Zusatzstoffe, wobei das Lösemittel (S) Folgendes ist:
- Entweder ein Keton mit der Formel (II) 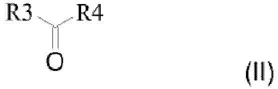 wobei R3 und R4 identisch oder verschieden sind, unabhängig voneinander ein Methylradikal, ein Ethylradikal, ein Isopropylradikal oder ein Isobutylradikal darstellen;
- oder eine Mischung, die für 100 Molprozent aus Folgendem besteht:
- Wasser in einem Verhältnis größer als 0 Molprozent und kleiner oder gleich 25 Molprozent; und
- ein Keton mit der Formel (II), wie oben definiert, in einem Verhältnis größer als oder gleich 75 Molprozent und kleiner 100 %;
einen Schritt b), im Laufe dessen die Polymerisationsreaktion durch Einführen in die Reaktionsmischung, die in Schritt a) hergestellt wurde,
eines Initiators mit freien Radikalen gestartet wird, dann bis zu seinem Abschluss der Entwicklung überlassen wird, um eine Ausfällung des linearen Polyelektrolyts zu erhalten.

12. Verwendung eines Polyelektrolyts, wie in einem der Ansprüche 1 bis 10 definiert, als Verdickungsmittel und/oder als Emulgator in kosmetischen oder pharmazeutischen Zusammensetzungen und insbesondere denjenigen, die für die Pflege und/oder die Konditionierung der Haare bestimmt sind.

13. Kosmetische oder pharmazeutische Zusammensetzung, **dadurch gekennzeichnet, dass** sie als Emulgator und/oder Verdickungsmittel eine wirksame Menge des Polyelektrolyts, wie in einem der Ansprüche 1 bis 10 definiert, enthält

## Claims

1. Linear, branched or cross-linked polyelectrolyte resulting from the polymerisation, for 100 mol %, of:
a) a molar ratio greater than or equal to 50% and less than or equal to 90% of monomer units originating from at least one cationic monomer,
b) a molar ratio greater than or equal to 1% and less than or equal to 50% of monomer units originating from at least one monomer comprising a fully or partially salified free weak acid function,
c) a molar ratio greater than or equal to 0.05% and less than or equal to 5 mol % of monomer units originating from at least one monomer of formula (I):
A-C(=O)-O-[(CH₂CH(R_{I})-O]ₙ-R (I)
wherein n represents a number between 4 and 25, A represents the vinyl radical (CH₂=CH-) or the isopropenyl radical [CH₂=C(CH3)-]
, R1 is a hydrogen atom, a methyl radical, or an ethyl radical, and R is a linear or branched, saturated or unsaturated aliphatic hydrocarbon radical having 8 to 30 carbon atoms, and
d) optionally a molar ratio greater than 0% and less than or equal to 1% of a diethylene or polyethylene cross-linking monomer.

2. Polyelectrolyte such as defined in claim 1, for which the monomer units originating from at least one cationic monomer, originate from the following quaternary ammonium salts:
- N,N,N-trimethyl 3-[(2-methyl 1-oxo 2-propenyl) amino] propanammonium salts;
- N,N,N-trimethyl 3-[(1-oxo 2-propenyl) amino] propanammonium salts;
- Diallyl dimethylammonium salts, or
- N,N,N-trimethyl 2-[(2-methyl 1-oxo 2-propenyl)] ethanammonium salts;

3. Polyelectrolyte such as defined in claim 2, for which the monomer units originating from at least one cationic monomer, originate from the following quaternary ammonium salts:
- N,N,N-trimethyl 3-[(2-methyl 1-oxo 2-propenyl) amino] propanammonium chloride;
- N,N,N-trimethyl 3-[(1-oxo 2-propenyl) amino] propanammonium chloride;
- Diallyl dimethylammonium chloride;
- N,N,N-trimethyl 2-[(1-oxo 2-propenyl)] ethanammonium chloride; or
- N,N,N-trimethyl 2-[(2-methyl 1-oxo 2-propenyl)] ethanammonium chloride.

4. Polyelectrolyte such as defined in claim 3, for which the monomer units originating from at least one cationic monomer, originate from the following quaternary ammonium salts:
- N,N,N-trimethyl 3-[(1-oxo 2-propenyl) amino] propanammonium chloride, or
- N,N,N-trimethyl 2-[(2-methyl 1-oxo 2-propenyl)] ethanammonium chloride.

5. Polyelectrolyte such as defined in any one of claims 1 to 4, for which the monomer units originating from at least one monomer comprising a fully or partially salified free weak acid function, originate from fully or partially salified free carboxylic acids:
- acrylic acid,
- methacrylic acid,
- itaconic acid,
- maleic acid, or
- 3-methyl 3-[(1-oxo 2-propenyl) amino] butanoic acid.

6. Polyelectrolyte such as defined in any one of claims 1 to 5, for which the compound having formula (I) such as defined hereinabove, is chosen from:
- pentacosaethoxylated behenyl methacrylate;
- tetraethoxylated lauryl acrylate;
- eicosaethoxylated stearyl methacrylate; or
- tetraethoxylated lauryl methacrylate;

7. Polyelectrolyte such as defined in any one of claims 1 to 6, resulting from the polymerisation, for 100 mol %, of:
a) a molar ratio greater than or equal to 60.00% and less than or equal to 90.00% of monomer units originating from at least one cationic monomer,
b) a molar ratio greater than or equal to 9.95% and less than or equal to 35.00% of monomer units originating from at least one monomer comprising a fully or partially salified free weak acid function, and
c) a molar ratio greater than or equal to 0.05% and less than or equal to 5.00 mol % of monomer units originating from the compound having formula (I) such as defined hereinabove.

8. Polyelectrolyte such as defined in claim 7, resulting from the polymerisation, for 100 mol %, of:
a) a molar ratio greater than or equal to 60.00% and less than or equal to 90.00% of monomer units originating from N,N,N-trimethyl 2-[(2-methyl 1-oxo 2-propenyl)] ethanammonium chloride,
b) a molar ratio greater than or equal to 9.95% and less than or equal to 35% of monomer units originating from fully or partially salified free acrylic acid and
c) a molar ratio greater than or equal to 0.05% and less than or equal to 5.00% of monomer units originating from pentacosaethoxylated behenyl methacrylate.

9. Polyelectrolyte such as defined in any one of claims 1 to 8, **characterised in that** it is cross-linked.

10. Polyelectrolyte such as defined in claim 9, resulting from the polymerisation, for 100 mol %, of:
a) a molar ratio greater than or equal to 60.00% and less than or equal to 90.00% of monomer units originating from N,N,N-trimethyl 2-[(2-methyl 1-oxo 2-propenyl)] ethanammonium chloride,
b) a molar ratio greater than or equal to 9.90% and less than or equal to 35% of monomer units originating from partially salified or free acrylic acid,
c) a molar ratio greater than or equal to 0.05% and less than or equal to 5.00% of monomer units originating from pentacosaethoxylated behenyl methacrylate. Et
d) a molar ratio greater than or equal to 0.005% and less than 1% trimethylol propanetriacrylate.

11. Method for preparing a polyelectrolyte such as defined in one of claims 1 to 10 comprising the following successive steps:
A step a) of preparing a reaction mixture comprising in the desired molar ratios and in a solvent (S), of monomer units originating from at least one cationic monomer, of monomer units originating from at least one monomer comprising a fully or partially salified free weak acid function, and optionally of monomer units originating from at least one monomer having formula (I) such as defined hereinabove and, if necessary or if desired of cross-linking agent and/or other additives,
said solvent (S) being:
- Either a ketone having formula (II): 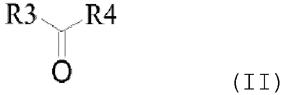 wherein R3 and R4 identical or different, represent independently from one another a methyl radical, an ethyl radical, an isopropyl radical or an isobutyl radical;
- Or a mixture consisting of, for 100 mol %:
- water in a ratio greater than 0 mol % and less than or equal to 25 mol %; and
- a ketone having formula (II) such as defined hereinabove, in a ratio greater than or equal to 75 mol % and less than 100%;
A step b) during which the polymerisation reaction is initiated by introduction into said reaction mixture prepared in step a), of a free radical initiator, then is allowed to unfold until the conclusion thereof, in order to obtain a precipitate of said linear polyelectrolyte.

12. Utilisation of the polyelectrolyte such as defined in one of claims 1 to 10, as a thickening agent and/or as an emulsifying agent in cosmetic or pharmaceutical compositions and more particularly those intended for the care and/or conditioning of hair.

13. Cosmetic or pharmaceutical composition **characterised in that** it contains as an emulsifying and/or thickening agent, an effective quantity of the polyelectrolyte such as defined in any one of claims 1 to 10.
